# EUROPÄISCHE PATENTANMELDUNG

(11) **EP 4 844 052 A1**
(43) Veröffentlichungstag der Anmeldung: **25.11.2026**
(21) Anmeldenummer: 26168221.5
(22) Anmeldetag: 26.03.2026
(51) Int. Cl.: C12M 1/107, C12M 1/00, C12M 1/12

(54) **BIO-ROHREAKTOR ZUR DURCHFÜHRUNG EINER GASFERMENTATION**

(30) Priorität: 20.05.2025 DE 102025119643
(71) Anmelder: FH Aachen, Körperschaft des öffentlichen Rechts, 52066 Aachen (DE)
(72) Erfinder: Hoffstadt, Kevin, 52428 Jülich (DE); Kuperjans, Isabel, 52072 Aachen (DE); Krafft, Simone, 52428 Jülich (DE)
(74) Vertreter: Michalski Hüttermann & Partner mbB

(57) **Zusammenfassung**

Die Erfindung betrifft einen Bio-Rohreaktor (1) zur Durchführung einer Gasfermentation unter Einsatz eines Substratgasgemisches (2) sowie von Mikroorganismen und/oder Enzymen in einer Flüssigphase zur Umsetzung des Substratgasgemisches in ein Produkt, mit
einem Reaktionsraum (3) zur Aufnahme der Flüssigphase und zur Durchführung der mikrobiellen bzw. enzymatischen Umsetzung, wobei der Reaktionsraum ein mehrfach gewickeltes Reaktionsrohr (4) aufweist,
einem Bubble-Generator (5) zum Erzeugen von Mikro- bzw. Nanobubbles aus dem Substratgasgemisch und zum Einbringen der Mikro- bzw. Nanobubbles in den Reaktionsraum und
einer Nährstoffzuführung (6) zum Zuführen von Nährstoffzusätzen.

Auf diese Weise wird ein Bio-Rohreaktor (1) zur Gasfermentation bereitgestellt, der einen effizienten Gas-Flüssig-Stoffübergang bei gleichzeitig hoher Prozessstabilität ermöglicht.

## Beschreibung

Die Erfindung betrifft einen Bio-Rohreaktor zur Durchführung einer Gasfermentation unter Einsatz eines Substratgasgemisches sowie von Mikroorganismen und/oder Enzymen in einer Flüssigphase zur Umsetzung des Substratgasgemisches in ein Produkt, mit einem Reaktionsraum zur Aufnahme der Flüssigphase und zur Durchführung der mikrobiellen bzw. enzymatischen Umsetzung.

Die Gasfermentation stellt eine vielversprechende biotechnologische Methode zur Gewinnung energiereicher Verbindungen dar. Je nach eingesetztem Mikroorganismus und Prozessführung können dabei verschiedene Produkte entstehen, darunter Methan, Essigsäure oder Alkohole. Im Fall der Methanbildung kann der erzeugte Biokraftstoff problemlos in bestehende Erdgasinfrastrukturen eingespeist, gespeichert und sektorübergreifend genutzt werden. Die Gasfermentation ist dabei ein biotechnologisches Verfahren, bei dem gasförmige Substrate - typischerweise Wasserstoff (H₂), Kohlendioxid (CO₂), Kohlenmonoxid (CO) oder Methan (CH₄) - von Mikroorganismen in wertvolle chemische Produkte umgewandelt werden. Die Mikroorganismen, meist anaerobe Bakterien oder Archaeen, nutzen diese Gase als Energie- und Kohlenstoffquelle, um beispielsweise Methan, Essigsäure, Ethanol oder andere organische Verbindungen zu produzieren.

Im Gegensatz zu klassischen Fermentationsverfahren, die auf gelösten organischen Substraten (wie Zucker oder Stärke) basieren, ist die Gasfermentation besonders interessant für die Nutzung von Industrieabgasen, Synthesegas oder überschüssigem Wasserstoff aus erneuerbaren Quellen. Sie bietet somit eine nachhaltige Möglichkeit zur CO₂-Nutzung und zur Herstellung von Biokraftstoffen und Plattformchemikalien. Der Reaktionsraum kann ein Bioreaktor sein, in dem die Mikroorganismen entweder als freie Zellen oder in Biofilmen wachsen und mit den gasförmigen Substraten über die Flüssigphase in Kontakt gebracht werden. Dabei ist ein effizienter Gas-Flüssig-Stoffübergang entscheidend für den Reaktionserfolg.

Trotz ihrer Vorteile - darunter milde Reaktionsbedingungen, hohe Toleranz gegenüber Verunreinigungen im Eduktgas und niedrige Prozesstemperatur im Vergleich zur chemisch-katalytischen Methanisierung - steht die biologische Methanisierung vor einer entscheidenden technischen Herausforderung: dem begrenzten Stoffübergang von Wasserstoff zwischen Gas- und Flüssigphase. Wasserstoff ist in Wasser nur sehr schlecht löslich. Daher stellt der Transport von gasförmigem H₂ in gelöster Form zur Zellmembran der Mikroorganismen einen Engpass dar, der die gesamte Reaktionskinetik und damit die Effizienz des Verfahrens limitiert. Konventionelle Ansätze zur Verbesserung des Gas-Flüssig-Stoffübergangs - wie mechanische Rührung, Gasrezirkulation oder Druckerhöhung - führen meist zu erhöhtem Energieverbrauch und bergen das Risiko, die empfindlichen Mikroorganismen durch Scherkräfte oder Druckschwankungen zu schädigen.

In diesem Kontext gewinnen sogenannte Nanobubbles, also Gasbläschen mit einem Durchmesser von weniger als 1.000 Nanometern, und Mikrobubbles, also Gasbläschen mit einem Durchmesser von weniger als 1.000 Mikrometern, zunehmend an Bedeutung. Aufgrund ihrer einzigartigen physikalischen Eigenschaften unterscheiden sich Mikro- und Nanobubbles grundlegend von herkömmlichen Makrobläschen. Sie besitzen eine enorm hohe spezifische Oberfläche und können aufgrund ihres geringen Auftriebs und der elektrostatischen Stabilisierung über lange Zeiträume stabil in Flüssigkeiten suspendiert bleiben. Während größere Gasblasen schnell aufsteigen und die Flüssigkeit verlassen, verbleiben Mikro- und Nanobubbles im Medium und bieten eine kontinuierliche Quelle für gelösten Wasserstoff. Diese langanhaltende Präsenz sowie die hohe Oberflächen-zu-Volumen-Ratio führen zu einem effizienteren Gasaustausch zwischen Blase und Flüssigkeit.

Besonders bemerkenswert ist, dass Mikro- und Nanobubbles beim Zerfall sogenannte "microstreaming"-Effekte hervorrufen können, bei denen kleine, lokale Flüssigkeitsbewegungen entstehen. Diese fördern die Konvektion und Durchmischung im Reaktor, ohne mechanische Rührwerke zu benötigen. Auf diese Weise kann der Stofftransport in der Umgebung der Mikroorganismen intensiviert werden, was den Wasserstofffluss zur Zellmembran erhöht. Zugleich wird durch die gleichmäßigere Verteilung der Bläschen im Medium eine Homogenisierung der Reaktionsbedingungen erreicht, was sich positiv auf die Prozessstabilität und die Produktbildung auswirkt. Durch den Einsatz von Mikro- und Nanobubbles kann die Methanproduktionsrate deutlich gesteigert und der Energieaufwand pro erzeugtem Kubikmeter Methan reduziert werden.

Es ist jedoch wichtig zu betonen, dass die Methanogenese nur eine von mehreren mikrobiellen Stoffwechselwegen ist, die in einem solchen gasbasierten Reaktionssystem ablaufen können. Die Auswahl und Aktivität der Mikroorganismen sowie die spezifischen Prozessbedingungen beeinflussen maßgeblich, welcher biochemische Weg im Vordergrund steht. Neben der Methanogenese kann unter bestimmten Bedingungen auch Homoacetogenese stattfinden. Dabei handelt es sich um einen mikrobiellen Prozess, bei dem Wasserstoff und Kohlendioxid durch homoacetogene Bakterien zu Essigsäure (Acetat) umgesetzt werden. Diese Bakterien gehören zu den anaeroben Mikroorganismen und konkurrieren in gemischten Kulturen oft mit Methanogenen um die gleichen Substrate, insbesondere Wasserstoff. Die Homoacetogenese ist dabei nicht nur eine alternative Verwertungsroute für H₂ und CO₂, sondern sie kann auch als Vorstufe oder Nebenweg in komplexeren mikrobiellen Netzwerken fungieren, etwa in Fermentations- oder syntrophen Konsortien.

Ein weiterer mikrobieller Prozess, der in gasfermentativen Systemen auftreten kann, ist die Solventogenese. Hierbei wandeln bestimmte anaerobe Mikroorganismen, vor allem Vertreter der Gattung Clostridium, einfache Substrate wie CO₂ und H₂ oder auch organische Zwischenprodukte in flüchtige organische Lösungsmittel wie Ethanol, Butanol oder Aceton um. Solventogene Stoffwechselwege finden vor allem in gemischten oder industriell gezüchteten Kulturen Anwendung, etwa zur Biotreibstoffproduktion. Auch diese Prozesse sind streng anaerob und auf eine ausreichende Versorgung mit gasförmigen Edukten angewiesen.

Die Methanogenese, Homoacetogenese und Solventogenese stellen somit drei exemplarische Stoffwechselwege dar, die in einem anaeroben, gasbasierten Reaktionssystem grundsätzlich ablaufen können. Welche dieser Reaktionen dominiert, hängt von zahlreichen Faktoren ab: darunter die Prozessbedingungen wie pH-Wert, Temperatur, Redoxpotenzial und Nährstoffversorgung, aber auch von der mikrobiellen Zusammensetzung des Biokatalysators. Der Reaktor muss folglich so ausgelegt sein, dass entweder gezielt eine gewünschte Reaktion gefördert oder unerwünschte Nebenreaktionen unterdrückt werden.

Ein entscheidender Hebel zur Steuerung der mikrobiellen Aktivität liegt in der effizienten Versorgung der Mikroorganismen mit den gasförmigen Substraten. An dieser Stelle setzt die Nanobubble-Technologie mit ihrem Potenzial zur Erhöhung des Gas-Flüssig-Stoffübergangs an. Durch die verbesserte Verfügbarkeit von Wasserstoff kann die Methanbildungsrate gesteigert werden, wodurch Reaktoren in deutlich kleineren Maßstäben bei gleichbleibender Leistung realisiert werden können. Zugleich eröffnet der Einsatz von Mikro- und Nanobubbles auch die Möglichkeit, Reaktoren gezielt für andere gasbasierte Stoffwechselwege zu optimieren, z. B. für die Produktion von Essigsäure oder Bioalkoholen.

Es ist die Aufgabe der Erfindung, einen Bio-Rohreaktor zur Gasfermentation bereitzustellen, der einen effizienten Gas-Flüssig-Stoffübergang bei gleichzeitig hoher Prozessstabilität ermöglicht.

Diese Aufgabe wird durch den Gegenstand des Patentanspruchs 1 gelöst. Bevorzugte Weiterbildungen finden sich in den Unteransprüchen.

Erfindungsgemäß wird somit ein Bio-Rohreaktor zur Durchführung einer Gasfermentation unter Einsatz eines Substratgasgemisches sowie von Mikroorganismen und/oder Enzymen in einer Flüssigphase zur Umsetzung des Substratgasgemisches in ein Produkt bereitgestellt, mit
einem Reaktionsraum zur Aufnahme der Flüssigphase und zur Durchführung der mikrobiellen bzw. enzymatischen Umsetzung, wobei der Reaktionsraum ein mehrfach gewickeltes Reaktionsrohr aufweist,
einem Bubble-Generator zum Erzeugen von Mikro- bzw. Nanobubbles aus dem Substratgasgemisch und zum Einbringen der Mikro- bzw. Nanobubbles in den Reaktionsraum und
einer Nährstoffzuführung zum Zuführen von Nährstoffzusätzen.

Bei der Gasfermentation besteht das Substrat aus einem Gasgemisch, das beispielsweise Wasserstoff und Kohlendioxid umfasst. Der Wasserstoff dient dabei als Energiequelle für die Mikroorganismen, während das Kohlendioxid als Kohlenstoffquelle genutzt wird. Diese gasförmigen Substrate werden von bestimmten Mikroorganismen unter anaeroben Bedingungen aufgenommen und in verschiedene chemische Produkte umgewandelt. So kann etwa durch Methanogenese Methan entstehen, während bei der Homoacetogenese Essigsäure gebildet wird. Im Fall der Solventogenese führen ähnliche Prozesse zur Bildung von Alkoholen wie Ethanol oder Butanol. Das Gasgemisch fungiert dabei als Rohstoff für die mikrobielle oder enzymatische Reaktion, bei der durch Fermentation ein gewünschtes Produkt entsteht. Da die Mikroorganismen in der wässrigen Flüssigphase aktiv sind, ist es entscheidend, dass das gasförmige Substrat effizient in diese Flüssigkeit überführt wird, um eine ausreichende Versorgung der Zellen mit den erforderlichen Reaktionspartnern sicherzustellen. Dies wird vorliegend durch die Mikro- bzw. Nanobubbles erreicht.

Ein besonderer Vorteil der Erfindung liegt in der Möglichkeit zur präzisen Steuerung der Gasverweilzeit, welche unmittelbar durch die Länge des Reaktionsrohres definiert ist. Hierdurch kann gezielt auf dynamische Prozessbedingungen reagiert werden, insbesondere auf Unterbrechungen oder Lastwechsel im Substratgasstrom. Die gezielte Einstellung der Verweilzeit trägt dazu bei, Lag-Phasen der mikrobiellen Kultur nach einer Unterbrechung zu minimieren und eine rasche Wiederaufnahme der Produktbildung, insbesondere einer Methanproduktion, zu ermöglichen. Darüber hinaus kann die Nährstoffzufuhr bedarfsgerecht an die aktuelle Produktbildung gekoppelt werden, wodurch eine kontinuierliche Versorgung der Mikroorganismen mit essenziellen Nährstoffkomponenten sichergestellt wird. Die Dosierung erfolgt über eine Nährstoffzuführung, die für verschiedene Nährstoffe separate Dosierpumpen aufweisen kann, sodass flexibel auf den Nährstoffbedarf reagiert werden kann. Gleichzeitig dient die gezielte Nährstoffzugabe auch dazu, dem im Prozess auftretenden Verdünnungseffekt durch die Bildung von Wasser entgegenzuwirken und eine stabile Prozessführung zu gewährleisten. Die Nährstoffe umfassen z. B. Stickstoff-, Phosphor-, Schwefelverbindungen sowie Spurenelemente

Ein weiterer Vorteil der Erfindung liegt im hohen erzielbaren Automatisierungsgrad, der eine kontinuierliche und dynamische Anpassung aller wesentlichen Prozessparameter in Echtzeit ermöglicht. Dazu zählen insbesondere die Regelung der Gas- und Flüssigströme sowie die bedarfsgerechte Nährstoffzufuhr. Durch diese umfassende Automatisierung wird eine konstante Prozessstabilität selbst unter wechselnden Betriebsbedingungen erreicht, was insbesondere für Anwendungen mit flexibler Fahrweise - etwa bei variabler Wasserstoffverfügbarkeit - von Vorteil ist. Gleichzeitig wird der Bedarf an manuellen Eingriffen deutlich reduziert, was sowohl die Betriebssicherheit erhöht als auch den Wartungsaufwand verringert. Die gewickelte Bauform des Rohrreaktors trägt zusätzlich zu einer verbesserten Durchmischung innerhalb der Flüssigphase bei und begünstigt zugleich den Eintrag und die Verteilung des Gases im Reaktionsraum. Dadurch werden die Voraussetzungen für einen effizienten Stoffaustausch und eine gleichmäßige mikrobielle Umsetzung des Substrats weiter verbessert.

Ein weiterer Vorteil der Erfindung liegt in der Reduktion mechanischer Belastungen, wie sie typischerweise in Systemen mit Rührwerken oder statischen Mischern auftreten. Die Reaktion erfolgt in einem strömungsbasierten System ohne bewegliche Mischelemente im Reaktionsraum, was die Belastung empfindlicher Mikroorganismen verringert und die mechanische Komplexität reduziert. Die Integration eines Bubble-Generators ermöglicht darüber hinaus eine effiziente Einbringung von Wasserstoff in die wässrige Phase bei gleichzeitig nur geringem Druckverlust. Da die Flüssigphase ohnehin im Kreislauf durch das System geführt wird, kann die vorhandene Pumpe genutzt werden, sodass der zusätzliche Energiebedarf infolge der Druckanforderung für den Bubble-Generator gering bleibt.

Zwar führt die Länge des gewickelten Reaktionsrohrs zu einem systemischen Druckverlust, dieser bewirkt jedoch zugleich einen erhöhten Druck an der Stelle der Gaseintragung.

Dadurch verbessert sich lokal die Löslichkeit von Wasserstoff, was den Gas-Flüssig-Stoffübergang gezielt begünstigt. Im Verlauf des Rohres sinkt der Druck allmählich wieder ab, wobei gleichzeitig auch der Wasserstoffanteil durch dessen Umsetzung zu Methan abnimmt. Die eingesetzte Energie wird somit effizient sowohl zur Erhöhung der Gaslöslichkeit als auch zur Förderung der Durchmischung genutzt.

Darüber hinaus erleichtert die Rohrkonfiguration die Wartung des Gesamtsystems. Im Gegensatz zu druckbeaufschlagten Rührkesselreaktoren oder Rieselbettreaktoren ist der Zugang zu funktionalen Komponenten wie Pumpe und Nanobubblegenerator einfacher möglich. Diese Bauteile können im Bedarfsfall durch kurzes Abschalten des Systems schnell und unkompliziert ausgetauscht werden. Auch die Einbindung von Bypass-Lösungen erlaubt einen sensorgestützten Betrieb mit der Möglichkeit, Sensoren im laufenden Prozess zu wechseln. Dies reduziert Stillstandszeiten und erhöht die Betriebssicherheit.

Ein zusätzlicher Vorteil ergibt sich durch die strömungsinduzierte Selbstreinigung des Rohrsystems: Die turbulente Strömung in Verbindung mit der abrasiven Wirkung der Mikro- bzw. Nanobubbles verhindert effektiv die Bildung und Anhaftung von Biofilmen, wodurch potenzielle Verstopfungen vermieden werden. Dadurch sinkt der Wartungsaufwand insgesamt und das System kann auch von weniger spezialisiertem Personal betrieben und instandgehalten werden.

Gemäß einer Weiterbildung der Erfindung weisen die in das Reaktionssystem eingebrachten Nanobubbles einen mittleren Durchmesser im Bereich zwischen 100 nm und 1000 nm auf und die Mikrobubbles weisen einen mittleren Durchmesser im Bereich von 1000 nm bis 1000 µm. Dieser Größenbereich ist besonders geeignet, um den Gas-Flüssig-Stoffübergang in der Flüssigphase signifikant zu verbessern. Durch ihre geringe Größe besitzen Mikro- bzw. Nanobubbles eine im Verhältnis zum Volumen sehr große spezifische Oberfläche, was die Kontaktfläche zwischen Gas und Flüssigkeit erhöht und somit die Diffusion der gasförmigen Substratkomponenten - insbesondere des schwer löslichen Wasserstoffs - in die Flüssigphase erleichtert. Zudem zeigen Mikro- bzw. Nanobubbles in diesem Größenbereich besondere physikalische Eigenschaften: Sie weisen eine hohe Stabilität in wässrigen Medien auf, verbleiben über längere Zeiträume homogen in Schwebe und steigen aufgrund ihres geringen Auftriebs nicht wie größere Blasen rasch zur Flüssigkeitsoberfläche auf. Dadurch wird nicht nur die Verweildauer des Gases in der Flüssigphase verlängert, sondern auch eine gleichmäßigere Verteilung des Substratgases innerhalb des Reaktionsraums erreicht. Dies führt insgesamt zu einer effizienteren Versorgung der Mikroorganismen mit den erforderlichen Reaktionspartnern und trägt dazu bei, die Umsatzrate des Fermentationsprozesses zu steigern. Darüber hinaus kann durch die Nutzung solcher Mikro- bzw. Nanobubbles der Energieeintrag für die Durchmischung reduziert werden, da eine gleichmäßige Gasverteilung auch ohne intensive mechanische Rührvorgänge erzielt wird. Der genannte Durchmesserbereich stellt somit einen funktionalen Kompromiss zwischen hoher Stoffübergangsrate, physikalischer Stabilität und technischer Erzeugbarkeit dar und unterstützt die Effizienz und Betriebssicherheit des Gesamtsystems wesentlich.

Gemäß einer Weiterbildung der Erfindung ist eine Druckpumpe vorgesehen, um den Bubble-Generator mit einem vorbestimmten Druck zu beaufschlagen. Diese Ausgestaltung ermöglicht eine gezielte Einstellung der für die Mikro- bzw. Nanobubble-Erzeugung erforderlichen Betriebsbedingungen, insbesondere bei der Verwendung hydrodynamischer Kavitationsverfahren, bei denen mittels des vorbestimmten Drucks stabile Mikro-bzw. Nanobubbles erzeugt werden. Die Druckpumpe gewährleistet dabei eine konstante Druckversorgung, unabhängig von Schwankungen im Systemdurchfluss oder von äußeren Einflüssen. Dadurch wird die Bildung von Mikro- bzw. Nanobubbles mit definierten Eigenschaften zuverlässig sichergestellt, was zu einer gleichmäßigen und effektiven Einbringung des Substratgases in die Flüssigphase führt. Ein weiterer Vorteil besteht darin, dass die Pumpe gleichzeitig zur Umwälzung der Flüssigphase genutzt werden kann, wodurch zusätzliche Bauteile entfallen und das System insgesamt kompakter und energieeffizienter gestaltet werden kann. Die gezielte Druckerzeugung an der Stelle der Gaseintragung führt darüber hinaus zu einer verbesserten Gaslöslichkeit, da mit steigendem Druck mehr Gas in der Flüssigkeit gelöst werden kann. Somit trägt die Druckpumpe in funktioneller Hinsicht zur Erhöhung der Gasverfügbarkeit, zur Verbesserung des Stoffübergangs und zur Stabilisierung des Prozesses bei. Sie stellt ein zentrales Element für die zuverlässige und leistungsfähige Umsetzung der gasförmigen Substrate im Reaktor dar. Der vorbestimmte Druck liegt vorzugsweise zwischen 0,4 bar und 4 bar. Ein Wert zwischen 1 und 2 bar, insbesondere 1,5 bar, hat sich als besonders geeignet herausgestellt.

Gemäß einer Weiterbildung der Erfindung weist das Reaktionsrohr eine Anzahl von Wicklungen auf, die zwischen 10 und 1.000 liegt. Diese gewickelte Anordnung des Rohrs bietet mehrere funktionale Vorteile für den Betrieb des Bio-Rohreaktors. Zum einen ermöglicht die Rohrwicklung eine platzsparende Realisierung einer langen Rohrstrecke innerhalb eines kompakten Bauvolumens, wodurch eine gezielte Einstellung der Verweilzeit des Reaktionsmediums erreicht werden kann. Die Verweilzeit ist ein entscheidender Parameter für die vollständige Umsetzung der gasförmigen Substrate durch die Mikroorganismen oder Enzyme in der Flüssigphase. Zum anderen fördert die gewundene Rohrführung die Durchmischung der Flüssigphase durch induzierte Sekundärströmungen und Turbulenzen. Dadurch verbessert sich die Homogenität innerhalb des Reaktionsraums, was wiederum zu einer gleichmäßigen Verteilung der Substrate und einer effektiveren mikrobiellen Umsetzung führt. Darüber hinaus trägt die hohe Rohrlänge, die durch die Vielzahl der Wicklungen realisiert wird, zur Druckerzeugung im System bei. Dieser Druck ist insbesondere im Bereich des Bubble-Generators von Vorteil, da hierdurch die Gaslöslichkeit erhöht und die Bildung stabiler Mikro- bzw. Nanobubbles begünstigt wird. Die Anzahl der Wicklungen kann dabei je nach Anwendungsfall und gewünschter Reaktorleistung angepasst werden, um ein optimales Verhältnis zwischen Verweilzeit, Stoffaustausch, Strömungsverhalten und Raumausnutzung zu erzielen.

Gemäß einer Weiterbildung der Erfindung liegt das Verhältnis von Länge zu Innendurchmesser des Reaktionsrohrs im Bereich zwischen 1.000 und 250.000. Dieses Verhältnis stellt einen wesentlichen Auslegungsparameter dar, der die Prozessbedingungen innerhalb des Bio-Rohreaktors maßgeblich beeinflusst. Durch ein solch hohes Längen-DurchmesserVerhältnis wird eine ausreichend lange Verweilzeit des Reaktionsmediums ermöglicht, was insbesondere bei der Umsetzung von gasförmigen Substraten mit geringer Löslichkeit - wie Wasserstoff - von entscheidender Bedeutung ist. Die relativ lange Rohrstrecke erlaubt es, die Mikroorganismen über einen ausgedehnten Zeitraum mit Substrat zu versorgen und so eine weitgehende Umsetzung zu erzielen, ohne dass dafür große Reaktorvolumina erforderlich wären. Gleichzeitig begünstigt das relativ enge Rohrquerschnittsprofil in Kombination mit der Länge die Ausbildung turbulenter Strömungsverhältnisse, wodurch sowohl die Durchmischung als auch der Stoffübergang zwischen Gas- und Flüssigphase verbessert werden können. Darüber hinaus erzeugt das hohe Verhältnis von Länge zu Durchmesser einen systemischen Druckverlust, der gezielt zur Druckerhöhung an der Stelle des Gaseintrags genutzt werden kann, was die Löslichkeit des Gases erhöht und die Effektivität der Mikro- bzw. Nanobubble-Erzeugung unterstützt. Das definierte Verhältnis von 1.000 bis 250.000 stellt dabei einen praxisgerechten Bereich dar, in dem eine gute Balance zwischen Reaktorgröße, Energieaufwand und Prozessleistung erzielt werden kann.

Gemäß einer Weiterbildung der Erfindung liegt der Innendurchmesser des Reaktionsrohrs zwischen 3 mm und 50 mm. Dieses Maß stellt einen entscheidenden Parameter für die Strömungsdynamik, die Verweilzeit und den Stoffübergang im Reaktor dar. Ein kleinerer Innendurchmesser führt zu einer höheren Strömungsgeschwindigkeit und begünstigt die Ausbildung turbulenter Strömungen, die wiederum zu einer verbesserten Durchmischung der Flüssigphase und zu einer effektiveren Verteilung der eingebrachten Mikro- bzw. Nanobubbles führen. Gleichzeitig wird dadurch die Kontaktfläche zwischen Gas und Flüssigkeit erhöht, was die Effizienz des Gas-Flüssig-Stoffübergangs deutlich steigert - ein zentraler Aspekt bei schwer löslichen Substraten wie Wasserstoff.

Ein größerer Innendurchmesser im oberen Bereich des genannten Bereichs ermöglicht hingegen die Verarbeitung höherer Volumenströme und kann bei einer entsprechenden Rohrlänge ebenfalls ausreichende Verweilzeiten gewährleisten. Die Bandbreite von 3 bis 50 mm erlaubt somit eine anwendungsflexible Auslegung des Reaktors, abgestimmt auf Prozessanforderungen wie Substratverfügbarkeit, gewünschte Produktionsleistung oder Skalierungsstufe. Darüber hinaus trägt die Begrenzung des Innendurchmessers zur Aufrechterhaltung eines moderaten Systemdrucks bei, was insbesondere im Zusammenspiel mit dem Bubble-Generator von Vorteil ist, da ein erhöhter Druck die Gaslöslichkeit unterstützt. Insgesamt unterstützt der definierte Innendurchmesserbereich die Effizienz, Skalierbarkeit und Betriebssicherheit des Reaktorsystems.

Gemäß einer Weiterbildung der Erfindung liegt die Länge des Reaktionsrohrs zwischen 20 und 2.000 Metern. Diese Rohrlänge ermöglicht eine gezielte Einstellung der Verweilzeit der Flüssigphase im Reaktor und bildet damit eine zentrale Stellgröße für die Reaktionskinetik des Gasfermentationsprozesses. Eine ausreichend lange Verweilzeit ist insbesondere bei biologischen Umsetzungen mit gasförmigen Substraten erforderlich, da Mikroorganismen oder Enzyme nur in begrenzter Geschwindigkeit aufgelöste Gase wie Wasserstoff und Kohlendioxid verarbeiten können. Durch die Wahl einer geeigneten Rohrlänge kann sichergestellt werden, dass auch bei schwankender Substratverfügbarkeit, beispielsweise in Folge einer diskontinuierlichen Wasserstoffversorgung, eine vollständige oder weitgehende Umsetzung erzielt wird. Darüber hinaus erzeugt eine größere Rohrlänge systembedingt einen erhöhten Druckverlust, der wiederum am Anfang des Systems zu einem leicht erhöhten Betriebsdruck führt. Dieser Effekt wird gezielt genutzt, um die Löslichkeit von Wasserstoff in der Flüssigphase zu verbessern und die Effektivität der Mikro- bzw. Nanobubble-Erzeugung zu steigern. Zudem begünstigt eine längere Rohrstrecke die Strömungsführung und die Durchmischung innerhalb des Systems, was wiederum zur Homogenität des Reaktionsmilieus und damit zur Prozessstabilität beiträgt. Die Auslegung der Rohrlänge im Bereich von 20 bis 2.000 Metern erlaubt eine bedarfsgerechte Anpassung an unterschiedliche Reaktorkapazitäten, Leistungsanforderungen oder Betriebsstrategien und erhöht dadurch die Anwendungsflexibilität des erfindungsgemäßen Reaktorsystems.

Gemäß einer vorteilhaften Weiterbildung der Erfindung ist zur dosierten Einbringung des Substratgasstroms ein Volumenstromregler vorgesehen. Durch den Einsatz eines solchen Reglers kann die Menge des in das Reaktorsystem eingebrachten Gasgemisches - z. B. bestehend aus Wasserstoff und Kohlendioxid - präzise gesteuert und an die aktuellen Prozessbedingungen angepasst werden. Dies ermöglicht eine bedarfsgerechte Substratversorgung der Mikroorganismen beziehungsweise Enzyme und trägt wesentlich zur Stabilität und Effizienz des Fermentationsprozesses bei. Insbesondere bei Prozessen mit diskontinuierlicher oder flexibler Gasbereitstellung, etwa bei elektrolytisch erzeugtem Wasserstoff aus erneuerbaren Energiequellen, ist eine genaue Regelung des Gasvolumenstroms entscheidend, um Schwankungen auszugleichen und eine gleichmäßige Reaktorbeladung sicherzustellen. Darüber hinaus ermöglicht der Volumenstromregler auch eine gezielte Steuerung der Reaktorverweilzeit des Gases, was wiederum die Effektivität des Gas-Flüssig-Stoffübergangs und die Ausnutzung des Substrats verbessert. Die Integration eines Volumenstromreglers stellt somit ein wichtiges Element zur prozesssicheren und energieeffizienten Betriebsweise des erfindungsgemäßen Bio-Rohreaktors dar.

Gemäß einer vorteilhaften Weiterbildung der Erfindung ist wenigstens ein Sensor zur Erfassung von Prozessparametern vorgesehen, wobei der Sensor aus der Gruppe bestehend aus Temperatur, pH-Wert, Gasvolumenstrom, Gaszusammensetzung und Methankonzentration ausgewählt ist. Die kontinuierliche Überwachung dieser Parameter ist entscheidend für eine stabile und effiziente Durchführung der Gasfermentation, da sie direkten Einfluss auf die Aktivität der eingesetzten Mikroorganismen oder Enzyme sowie auf die Reaktionskinetik des Gesamtprozesses haben. Die Temperatur und der pH-Wert bestimmen maßgeblich die Lebens- und Reaktionsbedingungen der biologischen Katalysatoren, während die Überwachung des Gasvolumenstroms und der Gaszusammensetzung eine genaue Steuerung der Substratversorgung erlaubt. Die Detektion der Methankonzentration im Produktgas ermöglicht darüber hinaus eine Echtzeitbewertung der Reaktorleistung und dient zugleich der Regelung der substrat- und nährstoffgekoppelten Zuführsysteme. Durch die Integration solcher Sensorik kann der Reaktor automatisiert betrieben und an wechselnde Betriebsbedingungen angepasst werden, wodurch Prozessstörungen frühzeitig erkannt und kompensiert werden können. Diese Ausgestaltung unterstützt somit nicht nur die Prozesssicherheit, sondern auch die Flexibilität und Wirtschaftlichkeit des Gesamtsystems.

Gemäß einer Weiterbildung der Erfindung ist ein Gasabscheider vorgesehen, um ein im Verlauf der Gasfermentation erzeugtes Produktgas von der flüssigen Reaktionsphase zu trennen. Diese Ausgestaltung ermöglicht die kontinuierliche oder diskontinuierliche Abführung des Produktgases - beispielsweise Methan - aus dem Reaktorsystem, ohne den Prozess zu unterbrechen oder die mikrobielle Aktivität zu beeinträchtigen. Der Gasabscheider ist dabei so ausgelegt, dass das entstandene Gas effizient aus dem Flüssigmedium entzogen werden kann, wodurch einerseits die Anreicherung unerwünschter Gasblasen im Reaktor vermieden wird und andererseits eine gezielte Analyse und Weiterverarbeitung des Produktgases ermöglicht wird. Durch die Trennung des Gases unmittelbar nach der Umsetzung im Reaktionsrohr kann außerdem der Reaktor entlastet und ein Rückstau oder Druckaufbau im System verhindert werden. Die Integration eines Gasabscheiders, z. B. in Form eines Zyklon-Gas-Flüssig-Separators, unterstützt somit eine kontinuierliche Prozessführung bei gleichzeitig hoher Produktqualität.

Nachfolgend wir die Erfindung anhand eines für die Erfindung nicht beschränkenden Ausführungsbeispiels unter Bezugnahme auf die Zeichnungen weiter im Detail erläutert.

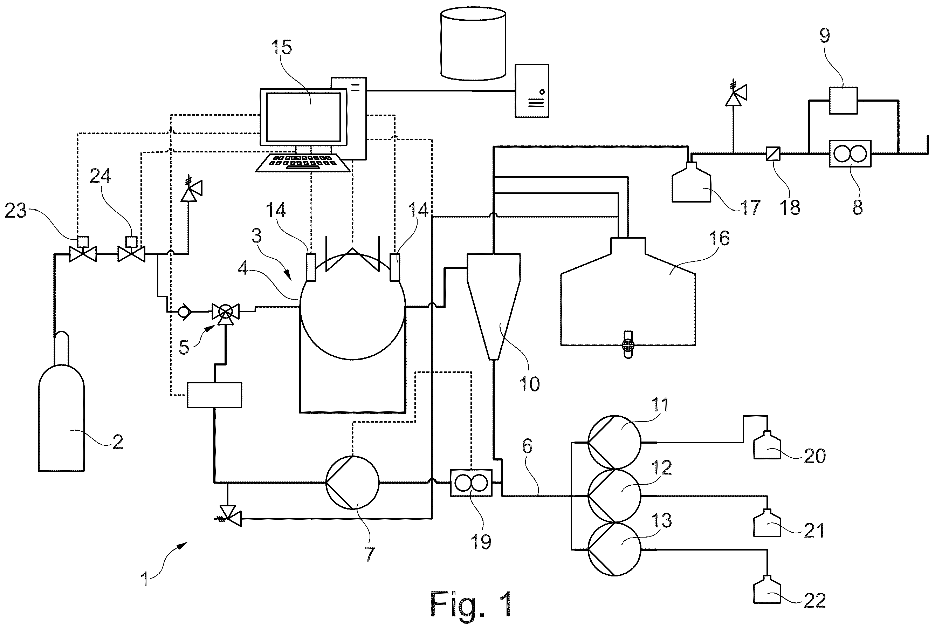

In der Zeichnung zeigt
- Fig. 1: schematisch eine Anordnung mit einem Bio-Rohreaktor gemäß einem Ausführungsbeispiel der Erfindung.

In der in Fig. 1 gezeigten Anordnung wird ein Bio-Rohreaktor 1 gemäß einem Ausführungsbeispiel der Erfindung betrieben, der für die Gasfermentation unter anaeroben Bedingungen ausgelegt ist. Als Substrat dient ein Gasgemisch 2 aus Wasserstoff und Kohlendioxid, das den Mikroorganismen in der Flüssigphase als Energie- bzw. Kohlenstoffquelle zur Verfügung gestellt wird. In dem Bio-Rohreaktor 1 gemäß dem hier beschriebenen Ausführungsbeispiel der Erfindung wird eine Methanogenese durchgeführt, bei der hydrogenotrophe Archaeen Methan aus den gasförmigen Substraten produzierten. Darüber hinaus sind prinzipiell auch weitere mikrobielle Prozesse wie die Homoacetogenese oder Solventogenese möglich, abhängig von den eingesetzten Gasen und Organismen und den Prozessbedingungen.

Der Bio-Rohreaktor 1 gemäß dem hier beschriebenen Ausführungsbeispiel der Erfindung besteht aus mehreren funktionalen Einheiten: Es ist ein Bubble-Generator 5 vorgesehen, über den das Gasgemisch 2 in Form ultrafeiner Gasblasen, sogenannten Mikro- bzw. Nanobubbles, mit einem Durchmesser von weniger als 1000 µm (Mikrobubbles) bzw. weniger als 1000 nm (Nanobubbles) in die Flüssigphase eingebracht wird. Die Erzeugung der Mikro-bzw. Nanobubbles erfolgt über ein hydrodynamisches Kavitationsverfahren in einem statischen Mischer. Zur Erzeugung der für diesen Vorgang erforderlichen Kavitationsbedingungen wird ein Systemdruck von etwa 1,5 bar verwendet, der im vorliegenden Aufbau durch eine Druckpumpe 7 erzeugt wird.

Die Flüssigphase, welche die aktiven Mikroorganismen oder Enzyme enthält, strömt durch ein spiralförmig gewickeltes Reaktionsrohr 4 mit einer Länge von 200 m und einem Innendurchmesser von 12 mm. Diese Rohrkonfiguration dient als Reaktionsraum 3, in dem die Umsetzung des Substratgasgemisches 2 stattfindet. Die Wicklung des Rohrs 4 erfüllt dabei mehrere Funktionen: Sie erlaubt die Realisierung einer langen Verweilzeit auf kleinem Raum, verbessert die Durchmischung infolge induzierter Sekundärströmungen und trägt durch die vergrößerte Oberfläche auch zur Wärmeabfuhr bei, was insbesondere bei exothermen Reaktionen von Vorteil ist. Die gewickelte Bauform des Reaktionsrohrs 4 begünstigt den Eintrag und die gleichmäßige Verteilung des Gasgemisches 2 in der Flüssigphase, was den Stoffaustausch und die mikrobielle Umsetzung des Substrats verbessert.

Der Druckabfall entlang des Rohrs 4 führt dazu, dass an der Stelle der Gaseintragung ein erhöhter Druck herrscht, der die Löslichkeit von Wasserstoff in der Flüssigphase verbessert. Gleichzeitig nimmt im Verlauf der Rohrstrecke die Wasserstoffkonzentration infolge der mikrobiellen Umsetzung kontinuierlich ab, sodass der Druckverlust im System zugleich effizient zur Stoffumsetzung genutzt wird. Das Reaktordesign ermöglicht zudem eine flexible Fahrweise, da nach Unterbrechungen eine rasche Wiederaufnahme der Methanproduktion erzielt werden kann.

Ein besonderer Vorteil besteht in der Selbstreinigung des Rohrsystems: Die turbulente Strömung in Verbindung mit der abrasiven Wirkung der Mikro- bzw. Nanobubbles verhindert effektiv die Bildung und Anhaftung von Biofilmen, wodurch potenzielle Verstopfungen vermieden werden. Dadurch sinkt der Wartungsaufwand insgesamt, und das System kann auch von weniger spezialisiertem Personal betrieben und instandgehalten werden. Auch der Zugang zu funktionalen Komponenten wie der Pumpe und dem Bubble-Generator 5 ist erleichtert. Diese lassen sich im Bedarfsfall durch kurzes Abschalten des Systems schnell austauschen.

Das System ist mit verschiedenen Sensoren ausgestattet, wie einem Sensor 8 zur Erfassung des Gasvolumenstroms und einem Sensor 9 zur Erfassung der Gaszusammensetzung. Die kontinuierliche Überwachung dieser Parameter trägt wesentlich zur Stabilität und Effizienz des Fermentationsprozesses bei. Darüber hinaus erfolgt die Zufuhr essenzieller Nährstoffe - darunter Stickstoff-, Phosphor- und Schwefelverbindungen sowie Spurenelemente und Natriumsulfid - aus Vorratsbehältern 20, 21, 22 über Dosierpumpen 11, 12, 13, die bedarfsabhängig aktiviert werden und auf eine gemeinsame Nährstoffzuführung 6 führen, die ihrerseits über einen Durchflussmesser 19 auf die Druckpumpe 7 geführt ist. Die Dosierung ist dabei an die Produktbildung gekoppelt, wodurch einerseits die Nährstoffversorgung der Mikroorganismen optimiert und andererseits ein Verdünnungseffekt infolge der Wasserbildung kompensiert wird.

Ein Volumenstromregler 23, dem ein Absperrventil 24 nachgeschaltet ist, sorgt für die dosierte Einbringung des Substratgasgemisch 2 in das System, was eine gezielte Steuerung der Gasverweilzeit ermöglicht. Diese Steuerung sowie auch die Steuerung einer optional einsetzbaren Temperierung 14 übernimmt eine zentrale Prozesssteuerung 15.

Die Reaktionsprodukte, insbesondere Methan, werden über einen nachgeschalteten Gasabscheider 10 kontinuierlich von der Flüssigphase getrennt. Dem Gasabscheider 10 sind ein Überlaufbehälter 16, eine Kondensatfalle 17 und ein Filter 18 nachgeschaltet. Die Produktqualität und -menge werden dabei fortlaufend mittels des Sensors 8 zur Erfassung des Gasvolumenstroms und des Sensors 9 zur Erfassung der Gaszusammensetzung überwacht.

Die dieser Patentanmeldung zu Grunde liegende Erfindung entstand in einem Projekt, welches unter dem Förderkennzeichen 03DPS1006 ("OptiFlexMethan") vom BMBF gefördert wurde.

### Bezugszeichenliste

- 1: Bio-Rohreaktor
- 2: Substratgasgemisch
- 3: Reaktionsraum
- 4: Reaktionsrohr
- 5: Bubble-Generator
- 6: Nährstoffzuführung
- 7: Druckpumpe
- 8: Sensor zur Erfassung des Gasvolumenstroms
- 9: Sensor zur Erfassung der Gaszusammensetzung
- 10: Gasabscheider
- 11: Dosierpumpe für Spurenelemente
- 12: Dosierpumpe für Makronährstoffe
- 13: Dosierpumpe für Natriumsulfid-Lösung
- 14: Temperierung
- 15: Prozesssteuerung
- 16: Überlaufbehälter
- 17: Kondensatfalle
- 18: Filter
- 19: Durchflussmesser
- 20: Vorratsbehälter für Spurenelemente
- 21: Vorratsbehälter für Makronährstoffe
- 22: Vorratsbehälter für Natriumsulfid-Lösung
- 23: Volumenstromregler
- 24: Absperrventil

## Patentansprüche

1. Bio-Rohreaktor (1) zur Durchführung einer Gasfermentation unter Einsatz eines Substratgasgemisches (2) sowie von Mikroorganismen und/oder Enzymen in einer Flüssigphase zur Umsetzung des Substratgasgemisches in ein Produkt, mit
einem Reaktionsraum (3) zur Aufnahme der Flüssigphase und zur Durchführung der mikrobiellen bzw. enzymatischen Umsetzung, wobei der Reaktionsraum ein mehrfach gewickeltes Reaktionsrohr (4) aufweist,
einem Bubble-Generator (5) zum Erzeugen von Mikro- bzw. Nanobubbles aus dem Substratgasgemisch und zum Einbringen der Mikro- bzw. Nanobubbles in den Reaktionsraum und
einer Nährstoffzuführung (6) zum Zuführen von Nährstoffzusätzen.

2. Bio-Rohreaktor nach Anspruch 1, wobei die Nanobubbles einen mittleren Durchmesser im Bereich zwischen 100 nm und 1000 nm und die Mikrobubbles einen mittleren Durchmesser im Bereich von 1000 nm bis 1000 µm aufweisen.

3. Bio-Rohreaktor nach Anspruch 1 oder 2, mit einer Druckpumpe (7) zum Beaufschlagen des Bubble-Generators mit einem vorbestimmten Druck.

4. Bio-Rohreaktor nach Anspruch 3, wobei der vorbestimmte Druck zwischen 0,4 bar und 7bar liegt.

5. Bio-Rohreaktor nach einem der vorhergehenden Ansprüche, wobei das Rohr (4) eine Anzahl von Wicklungen aufweist, die zwischen 10 und 1.000 liegt.

6. Bio-Rohreaktor nach einem der vorhergehenden Ansprüche, wobei das Verhältnis von Länge zu Innendurchmesser des Rohrs (4) zwischen 1.000 und 250.000 liegt.

7. Bio-Rohreaktor nach einem der vorhergehenden Ansprüche, wobei der Innendurchmesser des Rohrs (4) zwischen 3 und 50 mm liegt.

8. Bio-Rohreaktor nach einem der vorhergehenden Ansprüche, wobei die Länge des Rohrs (4) zwischen 20 und 2.000 m liegt.

9. Bio-Rohreaktor nach einem der vorhergehenden Ansprüche, mit wenigstens einem Sensor zur Erfassung von Prozessparametern ausgewählt aus Temperatur, pH-Wert, Gasvolumenstrom (8), Gaszusammensetzung (9) und Methankonzentration.

10. Bio-Rohreaktor nach einem der vorhergehenden Ansprüche, mit einem Gasabscheider (10) zum Trennen eines erzeugten Produktgases vom flüssigen Reaktionsmedium.
